Europäisches Patentamt

(19) European Patent Office

Office européen des brevets

(11) Publication number: **0 022 377**
**B1**

# (12) EUROPEAN PATENT SPECIFICATION

(45) Date of publication of patent specification: **10.04.85**

(21) Application number: **80302328.2**

(22) Date of filing: **09.07.80**

(51) Int. Cl.⁴: **G 01 N 33/52, A 61 B 10/00**

(54) **Device for obtaining stool samples.**

(30) Priority: **09.07.79 US 55636**

(43) Date of publication of application:
**14.01.81 Bulletin 81/02**

(45) Publication of the grant of the patent:
**10.04.85 Bulletin 85/15**

(84) Designated Contracting States:
**AT BE CH DE FR GB IT LI LU NL SE**

(56) References cited:
**AT-B- 356 825**
**US-A-3 965 888**
**US-A-3 996 006**

(73) Proprietor: **Levine, Robert Aaron**
**31 Pilgrim Lane**
**Guilford, Connecticut 06437 (US)**

(72) Inventor: **Levine, Robert Aaron**
**31 Pilgrim Lane**
**Guilford, Connecticut 06437 (US)**

(74) Representative: **Purvis, William Michael**
**Cameron et al**
**D. Young & Co. 10 Staple Inn**
**London WC1V 7RD (GB)**

Courier Press, Leamington Spa, England.

## Description

The invention relates to a stool sampling device which can be used by a patient, in the same manner as toilet tissue, to obtain a stool sample which can then be tested for signs of gastro-intestinal bleeding.

One medical procedure frequently used on physicians' patients involves the obtaining of a stool sample which is tested for traces of blood to determine the presence or absence of gastro-intestinal bleeding. This test is a conventional precaution for patients having an established history of gastro-intestinal bleeding and will also be used on patients who are anemic, and who complain of gastro-intestinal discomfort. This test is also used as a screening test during routine physical examinations.

The procedure most commonly used to obtain the stool sample is for the physician to don a rubber glove and manually insert a finger into the rectum of the patient to obtain a stool smear. The stool is then transferred to a piece of absorbent paper and effective amounts of detecting agents such as guaiac, ortho-tolidine, or ortho-dianisidine and hydrogen peroxide are applied to the stool whereupon the presence of blood in the stool will cause a bluish colouration to appear. No colour change indicates the absence of occult blood in the stool, and therefore, the absence of gastro-intestinal bleeding. This method of obtaining the stool sample is not sanitary and is unpleasant for the patient and for the physician.

Another method of obtaining the required stool samples involves the use of a kit sold under the brand name Hermoccult by Smith Kline Diagnostics. The kit includes a packet made of paper and formed somewhat similarly to a match book. The packet has a tab-slot interlock which can be opened so that the packet can be unfolded. Inside of the packet there is disposed a sample-receiving pad which has been treated with guaiac, one of the detecting chemicals referred to above. A sample-obtaining wooden stick is included with the kit. The kit is designed for use by the patient in privacy as follows. The kit is given to the patient by the physician, or obtained at a pharmacy upon a directive of the physician. The patient takes the kit home, and it is used subsequent to defecation. A scraping of the bowel movement is obtained by the patient from the toilet with the stick and some of the scraped material is transferred to the pad from the stick. The contaminated stick must then be discarded by the patient. The packet flap is then reclosed and the packet and sample are then returned to the physician's office for examination. It will be appreciated that this procedure is to some extent more desirable than the first above-described procedure in that it may be performed in privacy. Nevertheless, it is also un unclean procedure with no provision for guarding against contamination, and the manner of disposition of the specimen on the pad is somewhat distasteful in that the stool-contaminated pad is generally discarded in a waste bin in an unsealed state.

Other prior art stool sampling devices are disclosed in U.S. Patents Nos. 3 718 431 and 3 672 351.

U.S.A. specification 3 996 006 discloses a stool sampling device comprising a first folded over sheet of paper or cardboard with a pad secured between the folded together portions. The patient applies a stool smear to the pad through an opening in one of the portions using an applicator. A cover is then folded down to cover the opening and subsequently the stool smear can be tested by a physician by tearing an aperture in the other of the portions and applying reagents to the pad.

According to the invention there is provided a stool sampling device comprising a first sheet and absorbent means on the first sheet for receiving a stool smear characterised in that the first sheet is formed of pliant impermeable material; the absorbent means is formed as a pad which can receive a direct anal stool sample by drawing the pad across a patient's anus after defecation and said first sheet is foldable to contain said pad therewithin; a second sheet of pliant material is disposed on said first sheet, said second sheet overlying said one side of said first sheet and including an opening therethrough aligned with the pad so that a substantial area of said pad is exposed by said opening, first releasable adhesive means secure the second sheet to the first sheet, whereby the second sheet can be peeled off the first sheet after a stool smear has been deposited on the pad, and comprise means for hermetically sealing the pad within the first sheet after the stool smear has been deposited on the pad; an opening is provided in the first sheet, which opening is aligned with the pad when the pad is sealed within the first sheet, and the device includes a pliant impermeable cover sheet which hermetically seals the opening in the first sheet and is secured to the other side of the first sheet by second releasable adhesive means, the second adhesive means enabling the cover sheet to be peeled away from the first sheet to uncover the opening in the first sheet to expose the pad for the application of detecting reagents to the stool smear thereon, and enabling the cover sheet to be resealed to the first sheet after the stool smear has been tested.

Such a device can be used to procure stool samples, and which may be used in the same manner as toilet tissue is conventionally used after defecation, in the privacy of the patient's home or a toilet in a physician's clinic or office and can be clean and contamination-free. Thus the device can have a number of constituent layers, excess amounts of stool being removed by tearing off one of the constituent layers which is biodegradable and discarding that layer in the toilet. The portion of the device remaining is then hermetically sealed and returned to the physician's office and can be contamination and odour-free during transport. To examine the specimen for blood, the sealed package is opened and a reagent applied to the stool sample. Inspection for colour change is made, and the package is resealed and discarded. In this manner, the test can be performed quickly and the discarded package can be both contamination and odour-free.

The invention is diagrammatically illustrated by way of example in the accompanying drawings, in which:—

Figure 1 is an exploded perspective view of a preferred embodiment of a stool sampling device according to the invention;

Figure 2 is a perspective view of the device of Figure 1;

Figure 3 is a perspective view of the device of Figures 1 and 2 shown after a layer containing excess stool has been removed and discarded;

Figure 4 is a perspective view of the device of Figures 1 and 3 shown after a hermetic seal has been achieved to render the device contamination and odour-free; and

Figure 5 is a perspective view of the device showing how access to the stool sample is made for performing the test.

Referring to the drawings, a stool sampling device generally denoted 2, is a multi-layered assemblage which has the general pliancy and feel of a thickened piece of toilet tissue. An upper layer 4 is a sheet of semi-flexible material such as polyethylene plastic; paper-plastic laminate, or the like. In one half of the upper layer 4 there is an opening 6 and the half of the sheet 4 which contains the opening 6 has deposited thereon a layer 8 of soft fibrous cellulosic material which is flocked onto the sheet 4 and provides the flocked half thereof with a soft texture similar to tissue paper. Thus, one half of the sheet 4 will have a soft, tissue-like texture, and the other half will have a smooth, shiny texture, like plastic.

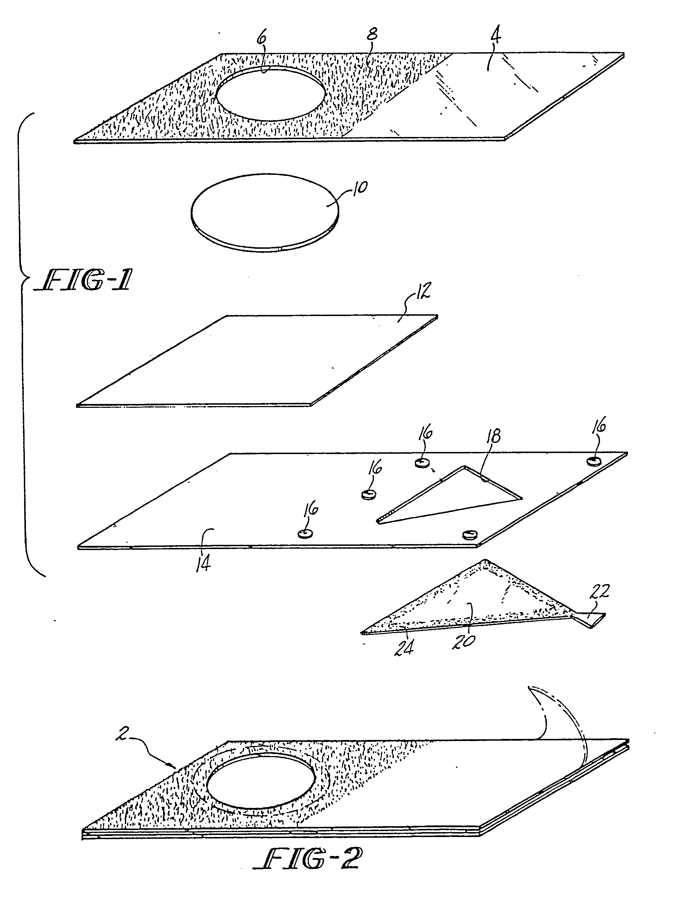

Disposed beneath the opening 6, there is a pad 10 somewhat enlarged compared with the opening 6, the pad 10 being of absorbent paper, or the like. An adhesive layer 12 underlies the pad 10 and serves to secure the pad 10 and the flocked half of the overlying sheet 4 to a bottom sheet 14, which is formed from an impermeable material such as a paper-plastic laminate, a foil-plastic laminate, or the like. The bottom sheet 14 is also semi-flexible so as to maintain the overall flexibility and pliability of the entire laminate. The layer 12 may take the form of a double-sided sticky tape, or may be simply a layer of sticky, resealable adhesive coated directly onto the sheet 14. If desired, and as shown in Figure 1, disposed on the half of the bottom sheet 14 not covered by the adhesive layer 12 can be a plurality of adhesive buttons 16 which serve releasably to adhere the non-flocked half of the sheet 4 to the sheet 14. The sheet 14 is provided with an opening 18 on one of its halves, and the opening 18 is closed and sealed by means of a conforming closure member 20 made from the same material as the sheet 14. The closure member 20 includes a pull tab 22 and a border 24 of resealable adhesive which serves to secure the closure member 20 to the sheet 14.

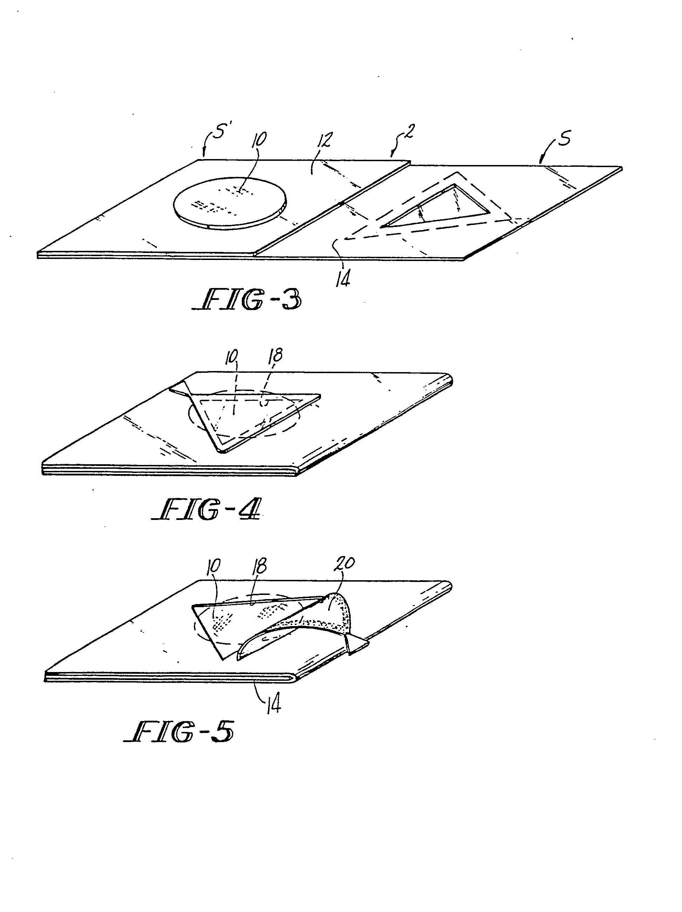

The appearance of the device 2 as dispensed by the physician, pharmacy, or the like, in its ready-to-use condition, is shown in Figure 2.

The device 2 is used to obtain a stool sample in the following manner. Immediately after defecation, the device is used in the same manner as one uses toilet tissue, and the flocked portion 8 of the device is drawn across the anus, whereby a stool smear is obtained on the flocked portion 8 and on the portion of the pad 10 which underlies the opening 6. The sheet 4 is then stripped away from the remaining portion of the device and discarded into the toilet to be flushed away with the stool. It will be noted that the adhesive buttons 16 will easily allow the half of the sheet 4 which overlies them to be removed from the sheet 14 (as shown partially in chain dotted lines in Figure 2) whereupon the remainder of the sheet 4 and the flocked portion 8 thereon will be peeled off the resealable adhesive layer 12.

After the sheet 4 has been removed from the device 2, the remaining portion of the device appears as shown in Figure 3. It will be noted that the pad 10, upon which stool has been deposited, remains adhered to the adhesive layer 12. The half S of the sheet 14 is then folded over on top of other half S' of the sheet 14 to form a pouch configured as shown in Figure 4. The folded-over half S is pressed against the exposed surface of the adhesive layer 12 so as hermetically to seal the resulting pouch whereby contamination and odour from the encased stool sample is prevented. It will be noted that the folding operation brings the sealed opening 18 into overlying relationship with the pad 10.

The sealed pouch is then delivered to the physician's office, testing laboratory, or the like, wherein the encased stool sample will be tested for occult blood. To expose the stool sample for testing, the closure member 20 is peeled back from the opening 18, as shown in Figure 5. The appropriate reagents are then applied to the stool sample on the pad 10. After the test has been performed, the closure member 20 is resealed over the opening 18 and the used pouch is discarded, in an hermetically sealed condition, for subsequent disposal, as by incineration or the like. It will be noted that, during the testing procedure, the underlying portion of the sheet 14 provides an impermeable barrier which prevents reagents, fecal material, viruses, bacteria, or the like from seeping through the pouch onto a laboratory bench.

It will be readily appreciated that a device according to the invention can provide means for obtaining a stool sample which is simple and natural to use, which can be used by a patient in privacy, and which circumvents the embarrassing aspects of the prior art devices and procedures. The sample, once obtained, can be hermetically sealed whereby contamination and odour problems are avoided. Still further, the device enables the actual test to be performed without reagent or contamination leakage occurring, and also enables the discarded tested stool sample to be contained in an hermetically sealed pouch.

**Claims**

1. A stool sampling device comprising a first sheet and absorbent means on the first sheet for receiving a stool smear characterised in that the first sheet (14) is formed of pliant impermeable material; the absorbent means is formed as a pad (10) which can receive a direct anal stool sample by drawing the pad (10) across a patient's anus after defecation and said first sheet (14) is foldable to contain said pad (10) therewithin; a second sheet (4) of pliant material is disposed on said first sheet (14), said second sheet (4) overlying said one side of said first sheet (14) and including an opening (6) therethrough aligned with the pad (10) so that a substantial area of said pad is exposed by said opening (6), first releasable adhesive means (12) secure the second sheet (4) to the first sheet (14), whereby the second sheet (4) can be peeled off the first sheet (14) after a stool smear has been deposited on the pad (10), and comprise means for hermetically sealing the pad within the first sheet (14) after the stool smear has been deposited on the pad (10); an opening (18) is provided in the first sheet (14), which opening (18) is aligned with the pad when the pad (10) is sealed within the first sheet (14), and the device includes a pliant impermeable cover sheet (20) which hermetically seals the opening (18) in the first sheet (14) and is secured to the other side of the first sheet (14) by second releasable adhesive means (24), the second adhesive means (24) enabling the cover sheet (20) to be peeled away from the first sheet (14) to uncover the opening (18) in the first sheet (14) to expose the pad (10) for the application of detecting reagents to the stool smear thereon, and enabling the cover sheet (20) to be resealed to the first sheet (14) after the stool smear has been tested.

2. A stool sampling device according to claim 1, characterised in that said means for hermetically sealing comprises a portion of said first releasable adhesive means (12).

3. A stool sampling device according to claim 1 or claim 2, characterised in that a portion (8) of the second sheet (4) is flocked with a layer of absorbent fibrous material in the area thereof surrounding the opening (6) in the second sheet (4).

4. A stool sampling device according to claim 1, characterised in that said first sheet (14) is elongate and has opposed lateral half portions; the pad (10) is situated on one of said half portions of the first sheet, the second sheet (4) overlies substantially all of said one side of the first sheet (14), the obverse surface of at least the portion of the second sheet (4) which overlies said one of said half portions of the first sheet (14) is flocked with a layer (8) of absorbent fibrous material; at least a portion of the first adhesive means (12) provides means for hermetically sealing the pad (10) within the first sheet (14) when the other of said half portions of the first sheet is folded over said one of said half portions of the first sheet after removal of the second sheet; and said opening (18) in the first sheet (14) is provided in said other half portion of the first sheet.

**Revendications**

1. Dispositif d'échantillonnage d'excréments comprenant une première feuille et des moyens absorbants sur cette première feuille pour recevoir une parcelle d'excrément, caractérisé en ce que la première feuille (14) est constituée en matériau imperméable pliable, les moyens absorbants sont constitués par une compresse (10) qui peut recevoir un échantillon anal direct d'excrément en faisant passer la compresse (10) à travers l'anus du patient après défécation, cette première feuille (14) étant pliable pour y renfermer ladite compresse (10); une seconde feuille (4) en matériau pliable est disposée sur cette première feuille (14), cette seconde feuille (4) recouvrant un premier côté de cette première feuille (14) et comprenant une ouverture (6) en regard de la compresse (10) de manière qu'une surface appréciable de cette compresse soit découverte par ladite ouverture (6), des premiers moyens adhésifs détachables (12) servent à fixer la seconde feuille (4) à la première feuille (14) de telle manière que la seconde feuille (4) puisse être détachée de la première feuille (14) après dépôt sur la compresse (10) d'une parcelle d'excrément, ce dispositif comprenant des moyens pour enfermer hermétiquement la compresse dans la première feuille (14) après que la parcelle d'excrément ait été déposée sur la compresse (10); un orifice (18) ménagé dans cette première feuille (14), en regard de la compresse (10) lorsque celle-ci est enfermée à l'intérieur de la première feuille (14), une feuille de couverture imperméable et pliable (20) bouchant hermétiquement l'orifice (18) de la première feuille (14) et fixée à l'autre côté de la première feuille (14) par des seconds moyens adhésifs détachables (24) permettant de détacher la feuille de couverture (20) de la première feuille (14) afin de dégager l'ouverture (18) de la première feuille (14) pour mettre à découvert la compresse (10) afin d'appliquer les réactifs de détection à la parcelle d'excrément qui s'y trouve et permettre à la feuille de couverture (20) d'être refixée sur la première feuille (14) après que la parcelle d'excrément ait été testée.

2. Dispositif d'échantillonnage d'excrément selon la revendication 1, caractérisé en ce que lesdits moyens pour enfermer hermétiquement la compresse comprennent une partie desdits premiers moyens adhésifs détachables (12).

3. Dispositif d'échantillonnage d'excréments selon la revendication 1 ou la revendication 2, caractérisé en ce qu'une partie (8) de la seconde feuille (4) est recouverte d'une couche de matériau fibreaux absorbant dans la partie de sa surface qui entoure l'ouverture (6) dans la seconde feuille (4).

4. Dispositif d'échantillonnage d'excréments selon la revendication 1, caractérisé en ce que cette première feuille (14) est de forme allongée et comporte deux moitiés latérales opposées, la compresse (10) est placée sur l'une de ces moitiés de la première feuille, la seconde feuille (4) recouvre sensiblement la totalité de ce côté de la première feuille (14) la surface opposée d'au moins une partie de la seconde feuille (4) qui recouvre ladite moitié de la première feuille (14) est recouverte d'une couche (8) de matériau fibreux absorbant, au moins une partie des premiers moyens adhésifs (12) permettent d'enfermer hermétiquement la compresse (10) à l'intérieur de la première feuille (14) quand l'autre moitié de la première feuille est repliée sur ladite première moitié de la première feuille après enlèvement de la seconde feuille; et ladite ouverture (8) de la première feuille est aménagée dans l'autre moitié de la première feuille.

## Patentansprüche

1. Vorrichtung zur Abnahme von Stuhlproben mit einem ersten Bogen und Absorbenseinrichtungen auf dem ersten Bogen zur Aufnahme von verschmiertem Stuhl, dadurch gekennzeichnet, daß der erste Bogen (14) von einem geschmeidigen undurchlässigen Material gebildet wird, die Absorbenseinrichtung als ein Kissen (10) ausgebildet ist, das eine direkte anale Stuhlprobe aufnehmen kann, indem das Kissen (10) nach Darmentleerung über den After eines Patienten gezogen wird, und der erste Bogen (14) so faltbar ist, daß er das Kissen (10) zwischen sich enthält, ein zweiter Bogen (4) aus geschmeidigem Material auf dem ersten Bogen (14) angeordnet ist, wobei der zweite Bogen (4) über der einen Seite des ersten Bogens (14) liegt und eine Öffnung (6) durch sich hindurch aufweist, die fluchtend mit dem Kissen (10) derart ausgerichtet ist, daß ein wesentlicher Bereich dieses Kissens unter der Öffnung (6) freiliegt, erste lösbare Hafteinrichtungen (12) den zweiten Bogen (4) an dem ersten Bogen (14) befestigen, wobei der zweite Bogen (4) von dem ersten Bogen (14) abgezogen werden kann, nachdem verschmierter Stuhl auf dem Kissen (10) aufgebracht wurde, und Einrichtungen zum hermetischen Abdichten des Kissens in dem ersten Bogen (14), nachdem der verschmierte Stuhl auf dem Kissen (10) aufgebracht wurde, haben, eine öffnung (18) in dem ersten Bogen (14) vorgesehen ist, die Öffnung (18) mit dem Kissen fluchtet, wenn das Kissen (10) in dem ersten Bogen (14) dicht eingeschlossen ist, und die Vorrichtung einengeschmeidigen undurchlässigen Deckbogen (20) aufweist, der die Öffnung (18) in dem ersten Bogen (14) hermetisch abschließt und auf der anderen Seite des ersten Bogens (14) mit Hilfe zweiter lösbarer Hafteinrichtungen (24) befestigt ist, wobei die zweiten Hafteinrichtungen (24) es ermöglichen, den Deckbogen (20) von dem ersten Bogen (14) abzuziehen und so die Öffnung (18) in dem ersten Bogen (14) aufzudecken und so das Kissen (10) für die Aufbringung von Nachweisreagentien auf dem verschmierten Stuhl darauf freizulegen, und es ermöglichen, den Deckbogen (20) wieder auf dem ersten Bogen (14) aufzusiegeln, nachdem der verschmierte Stuhl getestet wurde.

2. Vorrichtung zur Abnahme von Stuhlproben nach Anspruch 1, dadurch gekennzeichnet, daß die Einrichtungen zum hermetischen Abdichten aus einem Teil der ersten lösbaren Hafteinrichtung (12) bestehen.

3. Vorrichtung zur Abnahme von Stuhlproben nach Anspruch 1 oder Anspruch 2, dadurch gekennzeichnet, daß ein Abschnitt (8) des zweiten Bogens (4) mit einer Schicht von absorbierendem Fasermaterial in seinem Bereich, der die Öffnung (6) in dem zweiten Bogen (4) umgibt, beflockt ist.

4. Vorrichtung zur Abnahme von Stuhlproben nach Anspruch 1, dadurch gekennzeichnet, daß der erste Bogen (14) länglich ausgebildet ist und einander gegenüberliegende seitliche Hälften aufweist, das Kissen (10) auf einer dieser Hälften des ersten Bogens liegt, der zweite Bogen (4) im wesentlichen über der Gesamtheit dieser einen Seite des ersten Bogens (14) liegt, die Vorderseite wenigstens des Abschnittes des zweiten Bogens (4), der über der einen Hälfte des ersten Bogens (14) liegt, mit einer Schicht (8) von absorbierendem Fasermaterial beflockt ist, wenigstens ein Abschnitt der ersten Hafteinrichtung (12) Mittel zum hermetischen Abdichten des Kissens (10) in dem ersten Bogen (14) bildet, wenn die andere Hälfte des ersten Bogens über die eine Hälfte des ersten Bogens gefaltet ist, nachdem der zweite Bogen entfernt wurde, und die Öffnung (18) in dem ersten Bogen in der anderen Hälfte des ersten Bogens vorgesehen ist.

0 022 377

FIG-1

FIG-2

1

FIG-3

FIG-4

FIG-5